Europäisches Patentamt

European Patent Office

Office européen des brevets

(11) Veröffentlichungsnummer: **0 505 747 A2**

# EUROPÄISCHE PATENTANMELDUNG

(21) Anmeldenummer: **92103137.3**

(51) Int. Cl.5: **G01N 33/44**

(22) Anmeldetag: **25.02.92**

(30) Priorität: **23.03.91 DE 4109696**

(43) Veröffentlichungstag der Anmeldung:
**30.09.92 Patentblatt 92/40**

(84) Benannte Vertragsstaaten:
**AT CH DE ES FR GB IT LI SE**

(71) Anmelder: **Parker-Prädifa GmbH**
**Arnold-Jäger-Strasse 1**
**W-7120 Bietigheim-Bissingen(DE)**

(72) Erfinder: **Krumeich, Peter**
**Thomas-Mann-Strasse 21**
**W-7000 Stuttgart 30(DE)**

Erfinder: **Streit, Gerhard**
**Gerhard-Hauptmann-Weg 6/1**
**W-7101 Flein(DE)**
Erfinder: **Fuchs, Hans**
**Walsmünchener Strasse 6**
**W-8000 München 90(DE)**
Erfinder: **Strauss, Christian**
**Prälat-Pfanzelt-Strasse 1**
**W-8060 Dachau(DE)**

(74) Vertreter: **Wolff, Michael, Dipl.-Phys.**
**Kirchheimer Strasse 69 Postfach 750120**
**W-7000 Stuttgart 75(DE)**

(54) **Verfahren und Vorrichtung zum Bestimmen des Vulkanisationsgrades von Elastomeren.**

(57) **Gegenstand** der Erfindung sind ein Verfahren zum Bestimmen des Vulkanisationsgrades eines elastomeren Werkstücks und eine Vorrichtung zum Durchführen des Verfahrens.

**Stand der Technik** ist das Messen der Reißfestigkeit oder der Nadel-Eindringtiefe und das Errechnen des Vulkanisationsgrades aus dem Meßwert.

**Nachteil** ist die Beschränkung auf zeitlich umfangreiche Messungen an in größeren Abständen genommenen Stichproben. Eine Serienmessung ist ausgeschlossen.

**Aufgabe** ist die Angabe eines Verfahrens, bei welchem das Werkstück zerstörungsfrei bleibt und die Meßzeit so kurz ist, daß eine serienweise Durchführung möglich ist; und die Schaffung einer entsprechenden Vorrichtung.

**Lösung** sind ein Verfahren, bei dem das zu prüfende Werkstück verpreßt und plötzlich entlastet wird, worauf der Rückstellweg zeitlich verfolgt und daraus sein asymptotischer Wert ermittelt wird, aus dem nach Vorliegen des Ergebnisses der Bestimmung des bekannten Vulkanisationsgrades mindestens zweier Vergleichsproben der Vulkanisationsgrad des Werkstückes rechnerisch oder graphisch ermittelt wird; sowie eine Vorrichtung mit einer Laser-Optik zum Aufweiten, Kollimieren und Fokussieren eines Parallelstrahlenbündels, dessen Lichtfleck auf der Probenfläche optoelektrisch untersucht und mittels einer Objektiv-Nachführung konstant gehalten wird, wobei der Verlauf des Rückstellweges aufgenommen wird; insbesondere mit einer Werkstück-Justiereinrichtung (10), einer Verpreßeinrichtung (12), einer Weg/Zeit-Aufnahmeeinrichtung (14) und einer elektronischen Auswertungseinheit (16) mit Anwendungselektronik (18).

FIG.3

EP 0 505 747 A2

Die Erfindung betrifft ein Verfahren zum Bestimmen des Vulkanisationsgrades einer, insbesondere planparallelen, elastomeren Materialprobe eines Fertigteiles oder dieses selbst; sowie eine Vorrichtung zum Durchführen des Verfahrens. - Die Bestimmung des Vulkanisationsgrades dient hauptsächlich der Kontrolle einer vollständigen Vulkanisation, beispielsweise von in Serie gefertigten Dichtungsteilen.

Bei den bekannten stichprobenartigen Verfahren wird die Materialprobe einer Messung entweder der Reißfestigkeit oder der Eindringtiefe einer Nadel unterzogen, woraus jeweils der Vulkanisationsgrad ermittelt werden kann. Diese beiden bekannten Verfahren eignen sich offensichtlich nicht zur Serienbestimmung des Vulkanisationsgrades, weil im ersten Fall das Werkstück zerstört und im zweiten Fall wie im ersten die Bestimmung mit dem Herstellungstakt nicht Schritt hält.

Die Erfindung geht von der Erkenntnis aus, daß zwischen dem zu bestimmenden Vulkanisationsgrad eines Werkstückes einerseits und dessen Verhalten nach einer Verpressung des Werkstückes andererseits ein verifizierbarer gesetzmäßiger Zusammenhang besteht.

Der Erfindung liegt die Aufgabe Zugrunde, ein Verfahren zum Bestimmen des Vulkanisationsgrades einer elastomeren Materialprobe eines Fertigteiles oder dieses selbst anzugeben,bei welchem das Werkstück zerstörungsfrei bleibt und die Bestimmungszeit so kurz ist, daß eine serienweise Durchführung möglich ist.

Diese Aufgabe ist erfindungsgemäß dadurch gelöst, daß die Materialprobe bzw. das Fertigteil verpreßt, während einer Setzzeit unter, gegebenenfalls konstantem, Druck gehalten und anschließend plötzlich entlastet wird, worauf die Retardation der freigegebenen, gegebenenfalls ebenen, Probenfläche berührungslos gemessen wird, wobei

1.1) der bei der Retardation zurückgelegte Rückstellweg (S) einer ausgewählten Stelle der freigegebenen Probenfläche, oder eine seiner Ableitungen (Rückstellgeschwindigkeit bzw. -beschleunigung), in seiner Abhängigkeit von der Zeit (t) so lange aufgenommen wird, bis der asymptotische Wert ($S_\infty$) des Retardationsweges (S) für unendlich lange Aufnahmezeit ermittelt werden kann;

1.2) dieser asymptotische Wert ($S_\infty$) durch Anwendung der Gleichung:

$$S(t) = S_\infty \left(1 - e^{-\frac{t}{\tau_\sigma}}\right)$$

mit $\tau_\sigma$ als vulkanisationsgradabhängiger Retardationskonstanten ermittelt wird; und

1.3) der Vulkanisationsgrad (x) durch Anwendung der Gleichung:

$$X = \frac{\ln S_\infty - b}{a}$$

mit a und b als Konstanten, die aus der Bestimmung des bekannten Vulkanisationsgrades mindestens zweier Vergleichsproben mit gemäß Schritt 1.2 ermittelten $S_\infty$-Werten stammen, bestimmt wird.

Da dieses Verfahren zerstörungsfrei arbeitet und nur einen Zeitaufwand in der Größenordnung zehn Sekunden erfordert, löst es die gestellte Aufgabe.

Bei einer bevorzugten Durchführungsweise des erfindungsgemäßen Verfahrens wird nach Kompression der Materialprobe bzw. des Fertigteiles ein Strahlenbündel auf die freigegebene Probenfläche in deren ausgewählter Stelle fokussiert und der Fokus dieser wandernden Stelle nachgeführt; außerdem wird der Rückstellweg der ausgewählten Stelle der Probenfläche durch deren Verfolgung mittels des Fokus' des Strahlenbündels aufgenommen. Auf diese Weise ist das erfindungsgemäße Verfahren mit relativ einfachen Mitteln hinreichend genau durchführbar, falls der Durchmesser des Brennfleckes des fokussierten Strahlenbündels auf der ausgewählten Stelle des Werkstückes größenordnungsmäßig ein Mikrometer beträgt. Dabei läßt sich der Rückstellweg mit einer Genauigkeit von größenordnungsmäßig 0,1 Mikrometern verfolgen.

Bei der bevorzugten Durchführungsweise des erfindungsgemäßen Verfahrens wird Laserlicht verwendet und dessen Parallelstrahlenbündel aufgeweitet, kollimiert sowie fokussiert; außerdem wird das von der verfolgten Stelle der Probenfläche reflektierte Licht aus dem Strahlengang ausgespiegelt und in der Weise abgebildet, daß eine Vergrößerung des Lichtfleckes auf der verfolgten Stelle der Probenfläche in eine seitliche Verlagerung des abgebildeten Lichtfleckes umgewandelt wird; ferner wird diese Verlagerung gemessen und dementsprechend die Fokussierung korrigiert. Demnach handelt es sich hier um eine Regelung zur ständigen Aufrechterhaltung der Fokussierung, mit einer Meßkennlinie, das heißt einem Auflösungsvermögen, das je nach Reflektionsvermögen der verfolgten Stelle der Probenfläche ungefähr zwischen 2 Mikrometern für spiegelnde Oberflächen und 50 Mikrometern für schwach reflektierende Oberflächen liegt, beispielsweise bei stark streuenden (rauhen) oder absorbierenden (schwarzen) Oberflächen.

Der Erfindung liegt auch die Aufgabe zugrunde, eine Vorrichtung zum Durchführen des erfindungsgemäßen Verfahrens zu schaffen.

Diese Aufgabe ist gelöst durch die im direkten Strahlengang aufeinanderfolgenden Komponenten, nämlich:

01) einen Laser
02) eine Licht-Aufweitungsoptik
03) einen Kollimator

04) einen lichtdurchlässigen, auf seiner Rückseite verspiegelten, schräg gestellten Strahlenumlenker

05) ein Objektiv als Fühlglied eines Regelkreises

06) einen Träger der Materialprobe/des Fertigteiles

und durch die im senkrechten Strahlenabgang aufeinanderfolgenden Komponenten, nämlich:

07) eine Lichtfleck-Abbildungsoptik

08) ein Prisma

09) zwei nebeneinander angeordnete optoelektrische Sensoren

sowie durch

10) eine elektronische Anordnung als Regler zum Vergleich der Sensoren-Ausgangssignale und zum Steuern einer Objektiv-Nachführung

11) eine elektromechanische Objektiv-Nachführung als Stellglied des Regelkreises der zuvor genannten Komponenten: Objektiv/Lichtfleck-Abbildungsoptik/Prisma/Sensoren/Vergleichs- und Steuer-Anordnung/Objektiv-Nachführung/Objektiv

12) eine Gleichungs-Anwendungselektronik

Eine bevorzugte Ausführungsform der erfindungsgemäßen Vorrichtung zeichnet sich aus durch

1) eine Justiereinrichtung

2) eine Verpreßeinrichtung

3) eine Aufnahmeeinrichtung mit den Komponenten: Laser/Licht-Aufweitungsoptik/Kollimator/Strahlenumlenker/Objektiv/Lichtfleck-Abbildungsoptik/Prisma/Sensoren/gegebenenfalls Vergleichs- und Steuer-Anordnung/Objektiv-Nachführung

4) eine separate elektronische Auswertungseinheit mit der Anwendungselektronik

Bei der bevorzugten Ausführungsform ist als Träger der Materialprobe/des Fertigteiles ein Schlitten vorgesehen, welcher an der Verpreßeinrichtung vorbei zwischen der Justier- und der Aufnahmeeinrichtung bewegbar ist, und zwar entweder geradlinig oder auf einem Kreisbogen, der bei Erweiterung zum Kreis das gleichzeitige Bestücken und Durchführen des erfindungsgemäßen Verfahrens ermöglicht.

Bei der bevorzugten Ausführungsform ist die Aufnahmeeinrichtung zur Grobeinstellung des Objektivs insgesamt relativ zum Träger verstellbar angeordnet.

Statt der bevorzugten Durchführungsweise des erfindungsgemäßen Verfahrens (Anspruch 1) kann das in der Zeitschrift "Werkstoffe in der Fertigung 1-2/90" in dem Aufsatz "Optische Entfernungsmessung mit Laser/Optoelektronik in der dimensionalen Meßtechnik" erwähnte "Triangulationsverfahren" als bekannte optische Methode angewendet werden.

Im folgenden ist die Erfindung anhand der durch die Zeichnung beispielhaft dargestellten bevorzugten Ausführungsform der erfindungsgemäßen Vorrichtung zum Durchführen des erfindungsgemäßen Verfahrens zum Bestimmen des Vulkanisationsgrades von Elastomeren im einzelnen erläutert. Es zeigt:

Figur 1: Eine grafische Aufnahme und grafische Ermittlung gemäß Verfahrensschritt 1.1 bzw. 1.2

Figur 2: Eine grafische Bestimmung gemäß Verfahrensschritt 1.3

Figur 3: Eine schematische Darstellung der Vorrichtung

Figur 4: Eine schematische Darstellung der Aufnahmeeinrichtung der Vorrichtung.

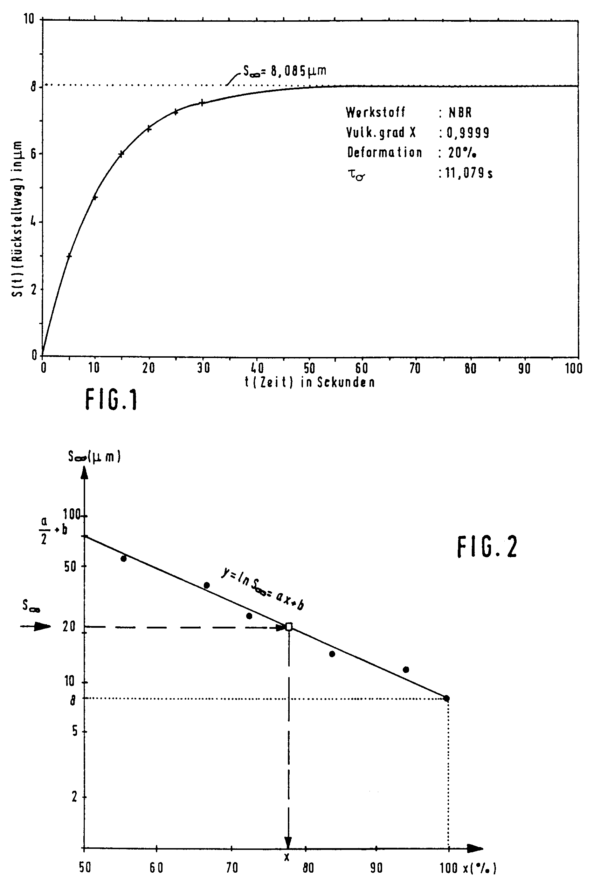

Wird bei einer zu 99,99% vulkanisierten Materialprobe aus dem Werkstoff NBR nach ihrer zwanzigprozentigen Verpressung alle fünf Sekunden der von der freigegebenen Probenfläche zurückgelegte Rückstell- oder Retardationsweg S (t) gemessen, dann ergeben sich innerhalb der Zeit t = 30 Sekunden sechs Wertepaare, die in Figur 1 durch Kreuze markiert sind und durch welche eine über den Zeitpunkt: 30 Sekunden nach Beginn der Messung hinaus extrapolierte Kurve gezogen wird, deren eingezeichnete Asymptote den Wert $S_\infty$ = 8,085 ergibt, wobei die Retardationskonstante $\tau_\sigma$ = 11,079s beträgt. Diese punktweise Aufnahme des bei der Retardation zurückgelegten Rückstellweges der ausgewählten Stelle der freigegebenen Probenfläche in seiner Abhängigkeit von der Zeit erfolgt also so lange, bis der asymptotische Wert des Retardationsweges für unendlich lange Aufnahmezeit: $S_\infty$ grafisch oder auch rechnerisch ermittelt werden kann.

Diese Ermittlung erfolgt grafisch bzw. rechnerisch durch Anwendung der Gleichung

$$S(t) = S_\infty(1 - e^{-\frac{t}{\tau_\sigma}}),$$

wobei die Retardationskonstante $\tau_\sigma$ vulkanisationsgradabhängig ist und bei der Berechnung automatisch anfällt.

Zur grafischen oder rechnerischen Bestimmung des gesuchten Vulkanisationsgrades X = 0,9999 durch Anwendung der Gleichung

$$X = \frac{\ln S_\infty - b}{a}$$

muß zunächst die Gerade $\ln S_\infty$ = aX + b festgelegt werden, und zwar nach Bestimmung des bekannten Vulkanisationsgrades mindestens zweier Vergleichsproben, deren asymptotische Werte $S_\infty$ wie gezeigt ermittelt wurden. Daraus ergeben sich die Gleichungskonstanten a (Steigung) und b (Ordinatenschnittpunkt). Anschließend kann nach Bildung des natürlichen Logarithmus' des asymptotischen Wertes $S_\infty$ der zugehörige Wert des gesuchten Vulkanisationsgrades abgelesen bzw. ausgerechnet werden. In Figur 2 ist ein Ablesebeispiel gegeben.

Das Verpressen und Unter-Druck-Halten währt je nach Elastizität des Werkstückes einige Sekunden oder einige zehn Sekunden.

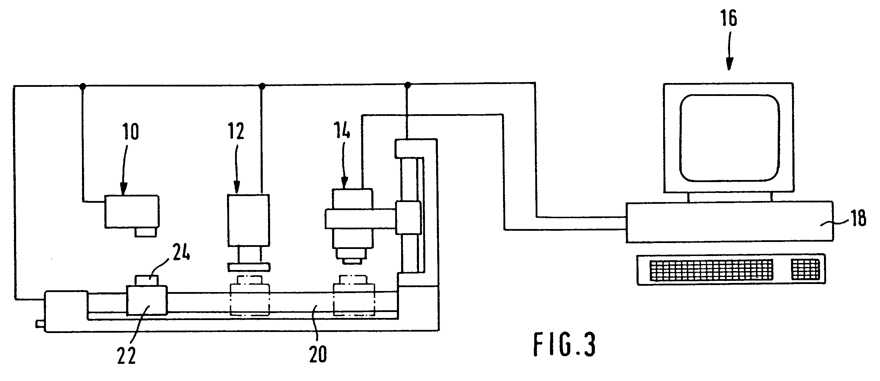

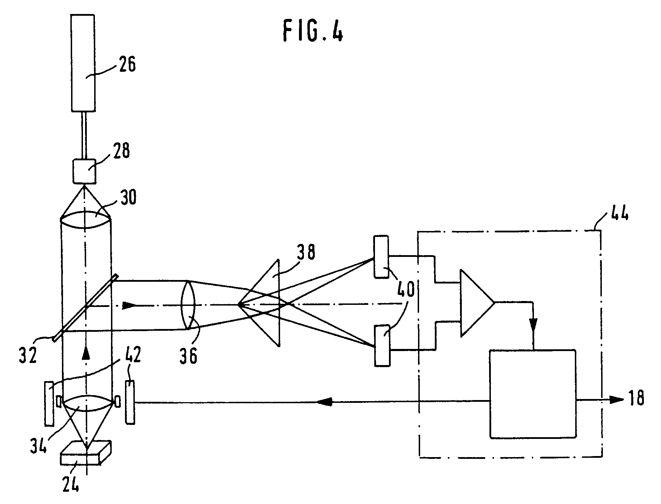

Nach Figur 3 besteht die erfindungsgemäße Vorrichtung im Ausführungsbeispiel aus einer Justiereinrichtung (10), einer Verpreßeinrichtung (12), einer Aufnahmeeinrichtung (14) und einer separaten elektronischen Auswertungseinheit (16) mit Gleichungs-Anwendungselektronik (18). Die drei Einrichtungen 10, 12 und 14 sind in gerader Reihe über einer geraden Schlittenführung (20) angeordnet, auf der ein Schlitten (22) als Träger eines Werkstückes (24) gleitet, bei dem es sich um eine Materialprobe eines Fertigteiles oder um dieses selbst handelt. Jenes mittels des bewegbaren Schlittens (22) transportierbare Werkstück (24) wird in der Justiereinrichtung (10) so ausgerichtet, daß die Flächennormale einer ausgewählten Stelle der zu belastenden Probenfläche vertikal steht. Danach wird die Probenfläche des Werkstückes (24) mittels der Verpreßeinrichtung (12) für eine gewisse Setzzeit unter Druck gebracht und gehalten sowie anschließend plötzlich entlastet. Schließlich wird die freigegebene Probenfläche, die sich schon während ihres Transportes zur Aufnahmeeinrichtung (14) auszudehnen begonnen hat, an ihrer ausgewählten Stelle optisch untersucht. Dazu ist die Aufnahmeeinrichtung (14) gemäß Figur 4 mit zwei senkrechten Strahlengängen ausgerüstet, nämlich einem vertikalen und einem in dessen Mitte abzweigenden waagrechten Strahlengang, die wie folgt gebildet sind:

Im vertikalen Strahlengang folgt von oben nach unten auf einen IR-Laser (26), der ein Parallelstrahlenbündel erzeugt, eine diese aufweitende Optik (28), ein das entstehende Kegelstrahlenbündel wieder parallelisierender Kollimator (30) (im einfachsten Fall eine Sammellinse), ein lichtdurchlässiger, auf seiner Rückseite verspiegelter, schräg gestellter Strahlenumlenker (32) und ein Objektiv (34) (im einfachsten Fall eine Sammellinse), welches das aufgeweitete Parallelstrahlenbündel auf die ausgewählte Stelle der Probenfläche des Werkstückes (24) fokussiert. Von jener möglichst reflektionsstarken Oberflächenstelle ausgehend, durchmißt das Licht den vertikalen Strahlengang in umgekehrter Richtung zurück bis zum Strahlenumlenker (32). Dort beginnt der waagrechte Strahlengang, in dem von links nach rechts auf den Strahlenumlenker (32) eine Optik (36) zur Abbildung des vom Werkstück (24) reflektierten Lichtfleckes (im einfachsten Fall eine Sammellinse), ein Prisma (38) und zwei nebeneinander angeordnete optoelektrische Sensoren (40) folgen. Diese Sensoren sind beispielsweise Fotodioden, die bei Fokussierung, das heißt minimalem Lichtfleckdurchmesser, gleich viel Licht erhalten, jedoch unterschiedlich viel Licht, wenn die ausgewählte Stelle der Probenfläche aus dem Fokus gewandert ist.

Damit das Objektiv (34) mittels einer elektromechanischen Nachführung (42) vertikal verstellt werden kann, um den Fokus mit der wandernden Stelle Schritt halten zu lassen und dadurch den oberflächlichen Lichtfleck minimal zu halten, werden die zwei elektrischen Ausgangssignale der beiden Sensoren (40) in einer elektronischen Anordnung (44) verglichen und anschließend zum Steuern der Objektiv-Nachführung (42) verwendet. Die elektronische Anordnung (44) ist also der Regler eines optisch-elektrisch-mechanischen Regelkreises mit dem Objektiv (34) als Fühlglied und der Objektiv-Nachführung (42) als Stellglied.

Mit diesem Regelkreis ist man in der Lage, die Fokussierung größenordnungsmäßig hundertmal pro Sekunde zu korrigieren.

Die elektronische Anordnung (44) ist im Ausführungsbeispiel Teil der Aufnahmeeinrichtung (14), könnte aber in die Gleichungs-Anwendungs-Elektronik (18) verlegt werden, die zur externen Auswertungseinheit (16) gehört und das elektrische Objektiv-Nachführungssignal zur Aufnahme des Rückstellweges der ausgewählten Stelle der Probenfläche empfängt. Natürlich sind als Signal auch die erste Ableitung (Geschwindigkeit) oder die zweite Ableitung (Verzögerung) des Rückstellweges = Objektiv-Nachführweges verarbeitbar.

Zum Einstellen des lichten Anfangsabstandes zwischen Objektiv (34) und Probenfläche des Werkstückes (24) auf ungefähr zwei Millimeter ist die Aufnahmeeinrichtung (14) als Ganze höhenverstellbar angeordnet, wobei ihr maximaler Meßbereich zur Aufnahme des Rückstellweges ungefähr einen Millimeter beträgt.

**Patentansprüche**

1.  Verfahren zum Bestimmen des Vulkanisationsgrades einer, insbesondere planparallelen, elastomeren Materialprobe eines Fertigteiles oder dieses selbst, dadurch **gekennzeichnet,** daß die Materialprobe bzw. das Fertigteil verpreßt, während einer Setzzeit unter, gegebenenfalls konstantem, Druck gehalten und anschließend plötzlich entlastet wird, worauf die Retardation der freigegebenen, gegebenenfalls ebenen, Probenfläche berührungslos gemessen wird, wobei

    1.1) der bei der Retardation zurückgelegte Rückstellweg (S) einer ausgewählten Stelle der freigegebenen Probenfläche, oder eine seiner Ableitungen, in seiner Abhängigkeit von der Zeit (t) so lange aufgenommen wird, bis der asymptotische Wert ($S_\infty$) des

Retardationsweges (S) für unendlich lange Aufnahmezeit ermittelt werden kann;

1.2) dieser asymptotische Wert (S∞) durch Anwendung der Gleichung:

$$S(t) = S_\infty (1 - e^{-\frac{t}{\tau_\sigma}})$$

mit $\tau_\sigma$ als vulkanisationsgradabhängiger Retardationskonstanten ermittelt wird; und

1.3) der Vulkanisationsgrad (X) durch Anwendung der Gleichung:

$$X = \frac{\ell n S_\infty - b}{a}$$

mit a und b als Konstanten, die aus der Bestimmung des bekannten Vulkanisationsgrades mindestens zweier Vergleichsproben mit gemäß Schritt 1.2 ermittelten S∞ - Werten stammen, bestimmt wird.

2. Verfahren nach Anspruch 1, dadurch gekennzeichnet, daß nach Kompression der Materialprobe bzw. des Fertigteils ein Strahlenbündel auf die freigegebene Probenfläche in deren ausgewählter Stelle fokussiert und der Fokus dieser wandernden Stelle nachgeführt wird; und daß der Rückstellweg der ausgewählten Stelle der Probenfläche durch deren Verfolgung mittels des Fokus' des Strahlenbündels aufgenommen wird.

3. Verfahren nach Anspruch 2, dadurch gekennzeichnet, daß Laserlicht verwendet und dessen Parallelstrahlenbündel aufgeweitet, kollimiert sowie fokussiert wird; daß das von der verfolgten Stelle der Probenfläche reflektierte Licht aus dem Strahlengang ausgespiegelt und in der Weise abgebildet wird, daß eine Vergrößerung des Lichtfleckes auf der verfolgten Stelle der Probenfläche in eine seitliche Verlagerung des abgebildeten Lichtfleckes umgewandelt wird; und daß diese Verlagerung gemessen und dementsprechend die Fokussierung korrigiert wird.

4. Vorrichtung zum Durchführen des Verfahrens nach Anspruch 3, gekennzeichnet durch die im direkten Strahlengang aufeinanderfolgenden Komponenten, nämlich:
    4.01) einen Laser (26)
    4.02) eine Licht-Aufweitungsoptik (28)
    4.03) einen Kollimator (30)
    4.04) einen lichtdurchlässigen, auf seiner Rückseite verspiegelten, schräggestellten Strahlenumlenker (32)
    4.05) ein Objektiv (34) als Fühlglied eines Regelkreises
    4.06) einen Träger (Schlitten 22) der Materialprobe/ des Fertigteiles (24)
und durch die im senkrechten Strahlenabgang aufeinanderfolgenden Komponenten, nämlich:
    4.07) eine Lichtfleck-Abbildungsoptik (36)
    4.08) ein Prisma (38)
    4.09) zwei nebeneinander angeordnete optoelektrische Sensoren (40)
sowie durch
    4.10) eine elektronische Anordnung (44) als Regler zum Vergleich der Sensoren-Ausgangssisgnale und zum Steuern einer Objektiv-Nachführung (42)
    4.11) eine elektromechanische Objektiv-Nachführung (42) als Stellglied des Regelkreises der Komponenten 05-07-08-09-10-11-05
    4.12) eine Gleichungs-Anwendungselektronik (18)

5. Vorrichtung nach Anspruch 4, gekennzeichnet durch
    5.1) eine Justiereinrichtung (10)
    5.2) eine Verpreßeinrichtung (12)
    5.3) eine Aufnahmeeinrichtung (14) mit den Komponenten 01 bis 05, 07 bis 09, gegebenenfalls 10,und 11
    5.4) eine separate elektronische Auswertungseinheit (16) mit der Anwendungselektronik (18)

6. Vorrichtung nach Anspruch 5, gekennzeichnet durch einen Schlitten (22) als Träger der Materialprobe/ des Fertigteiles (24), welcher an der Verpreßeinrichtung(12) vorbei zwischen der Justier- und der Aufnahmeeinrichtung (10 bzw.14) bewegbar ist.

7. Vorrichtung nach Anspruch 5 oder 6, dadurch gekennzeichnet, daß die Aufnahmeeinrichtung (14) zur Grobeinstellung des Objektivs (34) insgesamt relativ zum Träger (Schlitten 22) verstellbar angeordnet ist.

FIG.1

FIG. 2

FIG.3

EP 0 505 747 A2

FIG.4